# EUROPEAN PATENT SPECIFICATION

(11) **EP 4 425 297 B1**
(45) Date of publication and mention of the grant of the patent: **02.09.2026**
(21) Application number: 22887122.4
(22) Date of filing: 27.10.2022
(51) Int. Cl.: G05D 23/19, B01L 7/02, C12M 1/00

(54) **THERMOSTATIC APPARATUS, AND TEMPERATURE DISTRIBUTION IMPROVEMENT METHOD**
THERMOSTATISCHE VORRICHTUNG UND VERFAHREN ZUR VERBESSERUNG DER TEMPERATURVERTEILUNG
DISPOSITIF THERMOSTATIQUE ET PROCÉDÉ D'AMÉLIORATION DE LA RÉPARTITION DE LA TEMPÉRATURE

(30) Priority: 29.10.2021 JP 2021177376
(43) Date of publication of application: 04.09.2024
(73) Proprietor: Yamato Scientific Co. Ltd., Tokyo 103-0023 (JP)
(72) Inventor: YAMANASHI Akio, Tokyo 104-6136 (JP); OOHASHI Kazuki, Tokyo 104-6136 (JP)
(74) Representative: Grünecker Patent- und Rechtsanwälte PartG mbB
(86) International application number: PCT/JP2022/040148
(87) International publication number: WO 2023/074797

(56) References cited:
- EP-A1- 1 046 705
- JP-A- 2010 096 376
- JP-A- H03 262 940
- JP-U- S5 875 599
- KR-B1- 101 290 246
- US-A- 5 090 617
- US-A1- 2018 252 466

## Description

### TECHNICAL FIELD

The present invention relates to a thermostatic apparatus having a jacket structure formed by an inner tank and an outer tank, and a method of improving the temperature distribution of the thermostatic apparatus.

### BACKGROUND ART

Heretofore, thermostats having an air jacket structure formed by an inner tank and an outer tank have been proposed. This type of thermostat as a thermostatic apparatus is capable of accommodating and storing storage targets, such as chemicals, at constant temperatures.

This type of thermostat controls the temperature of an air layer (convection space layer) inside the outer tank to indirectly make the temperature inside the inner tank constant.

### CITATION LIST

### PATENT LITERATURE

Patent Literature 1: Japanese Utility Model Application Publication No. S58-75599
From document KR 101 290 246 B1 it is known a thermostatic apparatus according to the preamble portion of claim 1.
From document US 2018/252466 A1 it is known a freezer comprising a container/outer tank and an interior chamber/inner tank forming a jacket structure. A separator is arranged inside the outer tank and separating the jacket structure into a chamber.
From document EP 1 046 705 A1 it is known a thermostat/humidistat comprising an outer tank and an inner tank. A welded portion in the inner tank is welded by using an edge joint structure or butt-welded. The welded portion is provided at any position except corner portions. An interior housing is provided for use as a humidistat, incubator or food preserver. Furthermore, a housing structure includes steel sheet components joined together.
Document US 5 090 617 A refers to an automatic incubator comprising a chamber surrounded by a water jacket and insulation and provided with temperature and gas level sensors, a gas injection solenoid valve and separate water jacket and air heaters together with microprocessor proportional-integral-derivative control for regulating temperature and gas concentration within the chamber. A fan is provided to circulate gas within the chamber and distribute heat. Manual set controls for selecting the desired temperature and gas levels are provided. The automatic incubator comprises an outer tank and an inner tank.

### SUMMARY OF INVENTION

### PROBLEMS TO BE SOLVED BY INVENTION

Here, thermostats with the conventional air jacket structure, in particular, natural convection-type thermostats, control the temperature inside the inner tank through a layer of air flowing by convection through the space between the inner tank and the outer tank. For this reason, with the thermostats with the conventional air jacket structure, the convection in the air layer inside the air jacket structure may be affected by the power-on timing, the differences in temperature rise time between portions of the air jacket due to the structure of the air jacket, and the like. This may cause temperature unevenness inside the inner tank.

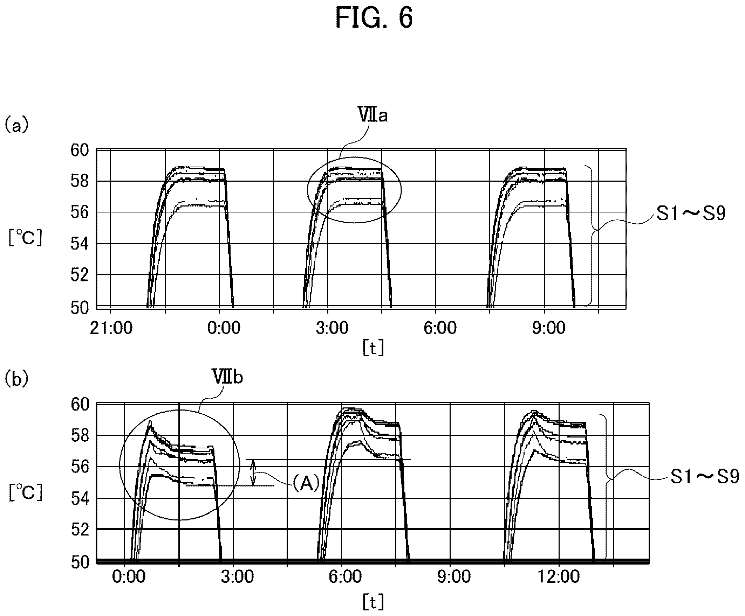

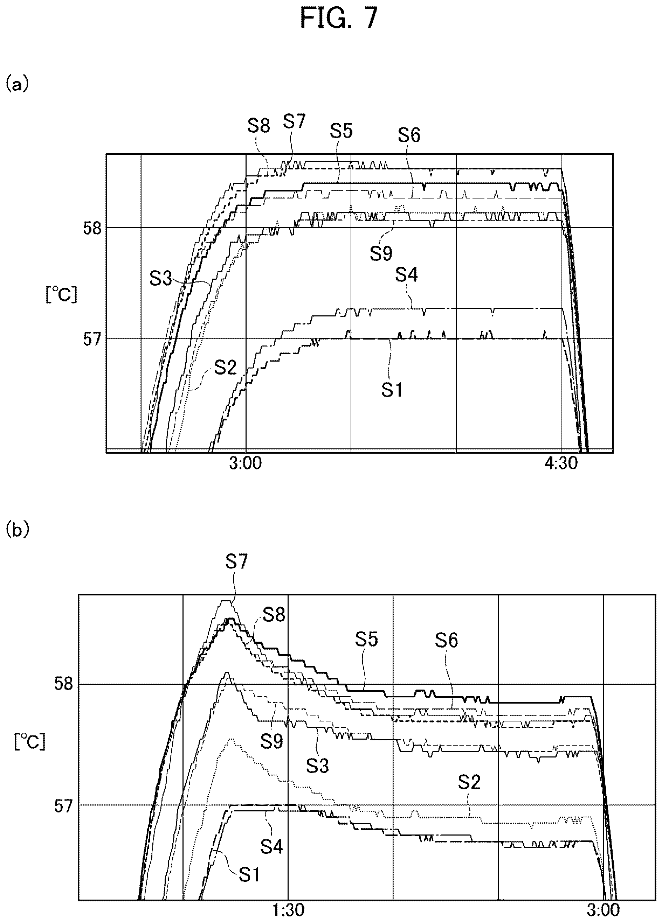

The temperature unevenness inside the inner tank can sometimes be a cause of a temperature shift in which the stabilized temperature shifts by about 1 to 2°C and a distortion phenomenon in which the temperature distribution (trend) changes. Here, the temperature shift is, for example, a later-described temperature shift A illustrated in Fig. 6(b). Also, the trend change is, for example, the later-described change to a temperature distribution illustrated in Fig. 7(b).

To address this, one may consider a method that involves forcibly circulating the air layer with a fan. However, installing a fan could be a cause of an increase in the cost of the thermostat, vibrations, or a deterioration in the temperature gradient.

An object of the present invention is to provide a thermostatic apparatus and a method of improving the temperature distribution of a thermostatic apparatus which are capable of reducing the occurrence of temperature unevenness inside the inner tank attributable to the power-on timing, the differences in temperature rise time between portions of the air jacket due to the structure of the jacket, and the like to reduce the temperature shift and the distortion phenomenon.

### SOLUTION TO SOLVE PROBLEMS

A thermostatic apparatus in accordance with an embodiment of the present invention includes the features of claim 1.

A temperature distribution improvement method of a thermostatic apparatus in accordance with an embodiment of the present invention is described in claim 8.

### EFFECTS OF INVENTION

With the above configurations, it is possible to provide a thermostatic apparatus and a method of improving the temperature distribution of a thermostatic apparatus which are capable of reducing the occurrence of temperature unevenness inside the inner tank attributable to the power-on timing, the differences in temperature rise time between portions of the jacket due to the structure of the jacket, and the like to reduce the temperature shift and the distortion phenomenon.

### BRIEF DESCRIPTION OF DRAWINGS

[Fig. 1] Fig. 1 is a perspective view illustrating a schematic configuration of a thermostatic apparatus according to one embodiment.
[Fig. 2] Fig. 2 illustrates the schematic configuration of the thermostatic apparatus illustrated in Fig. 1, with Fig. 2(a) being a partial cross-sectional view of the thermostatic apparatus as seen from the front side, and Fig. 2(b) being an enlarged view illustrating a part taken out of Fig. 2(a).
[Fig. 3] Fig. 3 is a partial cross-sectional view of the thermostatic apparatus illustrated in Fig. 1 as seen from one lateral side, illustrating the schematic configuration of the thermostatic apparatus.
[Fig. 4] Fig. 4 is a schematic view presented to explain a heating method of the thermostatic apparatus illustrated in Fig. 1.
[Fig. 5] Fig. 5 is a schematic view for explaining a temperature detection method of the thermostatic apparatus according to the one embodiment.
[Fig. 6] Fig. 6(a) is a graph exemplarily illustrating a result of temperature detection of the thermostatic apparatus according to the one embodiment, and Fig. 6(b) is a graph exemplarily illustrating a result of temperature detection of a conventional apparatus.
[Fig. 7] Fig. 7(a) is an enlarged diagram of the part indicated as Vlla in Fig. 6(a), and Fig. 7(b) is an enlarged diagram of the part indicated as Vllb in Fig. 6(b).
[Fig. 8] Fig. 8 is a perspective view illustrating a schematic configuration of a thermostatic apparatus according to another embodiment.

### DESCRIPTION OF EMBODIMENTS

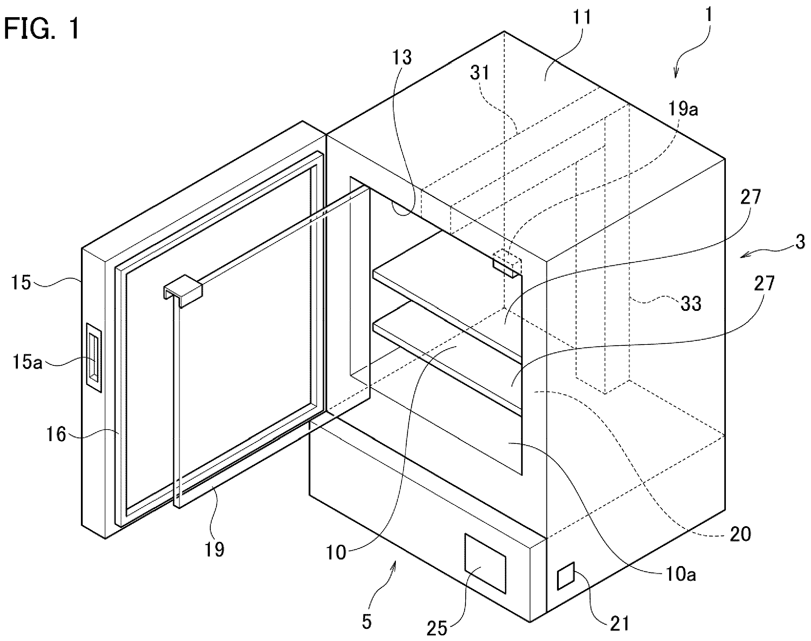

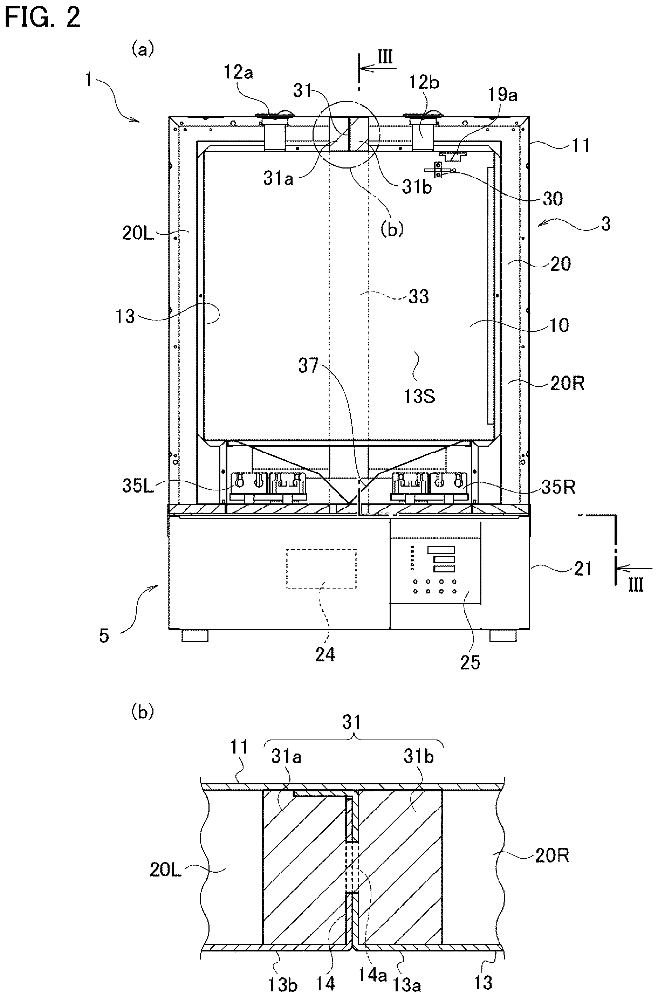

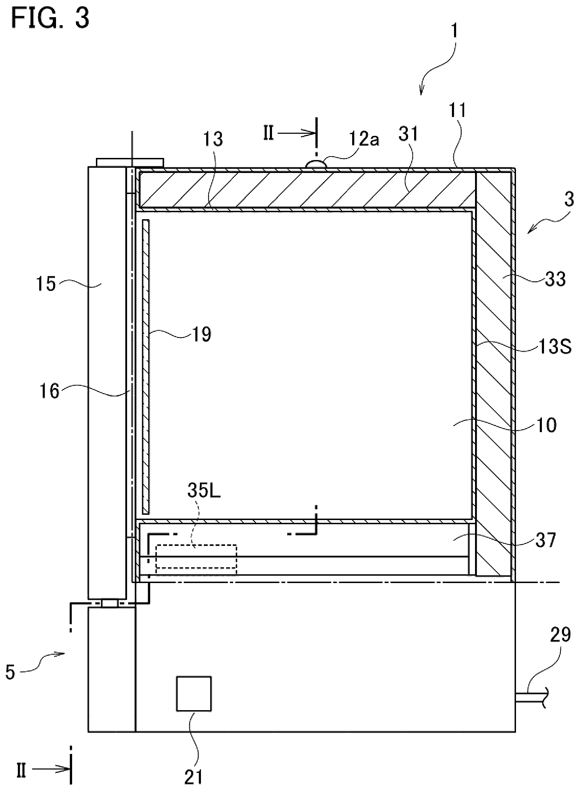

Figs. 1 to 3 illustrate an example configuration of a thermostatic apparatus 1 according to one embodiment. Note that Fig. 1 is a perspective view illustrating the thermostatic apparatus 1 with an outer door 15 partly opened. Fig. 2(a) is a partial cross-sectional view of the thermostatic apparatus 1 along the II-II line in Fig. 3 as seen from the front side in the front-rear direction. Fig. 2(b) is an enlarged view illustrating the circled part (b) in Fig. 2(a). Fig. 3 is a partial cross-sectional view of the thermostatic apparatus 1 along the III-III line in Fig. 2 as seen from one lateral side in the right-left direction. Here, the front-rear direction refers to the depth direction of the thermostatic apparatus 1, and the right-left direction refers to the width direction of the thermostatic apparatus 1.

As illustrated in Figs. 1 to 3, the thermostatic apparatus 1 includes a body 3 and a control device 5 provided under the body 3. As illustrated in Fig. 2(a), the control device 5 includes an operation panel 25 at the front surface and a control board (controller) 24 and the like on the inside. As illustrated in Fig. 3, the control device 5 also includes an outlet plug 29 at the back surface. Incidentally, an external terminal 21 and the like can arranged at one lateral surface of the control device 5.

The body 3 includes, for example, an outer tank 11 made of stainless steel and an inner tank 13 made of stainless steel which is provided inside the outer tank 11. The inner tank 13 is a constant temperature chamber 10 to accommodate and store storage targets, such as chemicals, for example.

Specifically, the thermostatic apparatus 1 includes an outer tank 11 and an inner tank 13 which is provided inside the outer tank 11 and forms a jacket structure with the space between itself and the outer tank 11. In the present embodiment, the jacket structure is an air jacket, and controls the temperature of an air layer 20 between the outer tank 11 and the outer peripheral surface of the inner tank 13 to maintain the temperature of the constant temperature chamber 10 inside the inner tank 13 uniform. The thermostatic apparatus 1 is, for example, a natural convection-type incubator. The air layer 20 may be rephrased as a convection space layer or a heat flow circulation area.

As illustrated in Fig. 1, the body 3 includes the outer door 15, which is openable and closable toward and from the front side. The body 3 includes an inner door 19 made of tempered glass which is provided inward of the outer door 15 and through which the inside of the constant temperature chamber 10 can be observed. The outer door 15 is a door with a lock, for example. Since the outer door 15 can be locked, the security of the constant temperature chamber 10 is ensured. The inner door 19 is configured to be openable and closable alone. In this way, it is possible to easily observe the inside of the constant temperature chamber 10 without the inner door 19 causing an abrupt temperature change when the outer door 15 is opened. Moreover, this configuration of the inner door 19 makes it possible to easily put storage targets into and out of the constant temperature chamber 10.

Here, the body 3 has an opening portion 10a at the one surface where the outer door 15 and the inner door 19 are provided. When the body 3 is closed, the inner door 19 and the outer door 15 close the opening portion 10a. A magnet packing 16 is arranged on the back portion of the outer door 15 which faces the periphery of this opening portion 10a in the closed state, in consideration of the vibrations at the time of opening and closing the outer door 15. Specifically, the outer door 15 is provided with the magnet packing 16 as a sealing member at the position that comes into contact at least with the above-mentioned one surface of the body 3. When the outer door 15 is closed, the outer door 15 and the one surface squish the magnet packing 16, thereby tightly closing the constant temperature chamber 10. Incidentally, at the lateral surface of the outer door 15 on its opening and closing end, there is provided a handle portion 15a for the user to hook their hand (fingers) at the time of opening and closing it.

On the other hand, in the constant temperature chamber 10, a plurality of (e.g., two) shelf boards 27 are arranged whose positions in the up-down direction are freely changeable according to the sizes of the storage targets and the like. Also, a magnet-type attachment-detachment member 19a for keeping the inner door 19 closed when it is closed is arranged at an upper edge portion of the opening portion 10a. The inner door 19 is configured to maintain the opening portion 10a closed with the magnetic force of the attachment-detachment member 19a. The inner door 19 is configured such that, as the user pushes in the inner door 19 when opening the inner door 19, the inner door 19 gets released from the attachment-detachment member 19a and opens outward.

As the outer door 15 and the inner door 19 close the opening portion 10a as described above, the constant temperature chamber 10 is formed inside the inner tank 13.

Note that a temperature detector 30 that detects the temperature of the constant temperature chamber 10 is provided inside the inner tank 13, as illustrated in Fig. 2(a). Information of the temperature detected by this temperature detector 30 is provided to the control board 24 of the control device 5. The control board 24 controls later-described heaters 35L and 35R based on this temperature information provided from the temperature detector 30 to adjust the temperature of the air layer 20.

Also, through-holes 12b for ventilating the constant temperature chamber 10 are formed in the upper surface of the outer tank 11. Moreover, opening-closing lids 12a for opening and closing the through-holes 12b are provided on the upper surface of the outer tank 11.

As illustrated in Fig. 2(a), for example, the inner tank 13 is formed of a thin plate of stainless steel folded in a rectangular shape. As illustrated in Figs. 2(a) and 2(b), the inner tank 13 has a first portion 14 formed by partly joining the above thin plate's opposite end portions 13a and 13b around the center position on a top surface portion of the inner tank 13 in the right-left direction. As illustrated in Fig. 2(b), the configuration of the first portion 14 is such that the opposite end portions 13a and 13b are partly joined to each other with an opening portion (penetrating portion) 14a formed in part of the first portion 14. Moreover, the end portion 13a on one side of the first portion 14 extends toward the outer tank 11. The one end portion 13a extending toward the outer tank 11 is folded in an L-shape to support the inner surface of the outer tank 11. Note that, as illustrated in Fig. 1, one plane (front plane) of the inner tank 13 is open, thereby forming the opening portion 10a of the constant temperature chamber 10. The opposite plane (rear plane) of the inner tank 13 is closed by a thin plate 13S of stainless steel.

This thermostatic apparatus 1 includes a separator including a horizontal heat insulation member 31 and a vertical heat insulation member 33, for example. As illustrated in Figs. 1, 3, etc., in the thermostatic apparatus 1, the air layer 20 in the air jacket structure is divided and separated into a left air layer 20L and a right air layer 20R which are laterally symmetrical to each other (divided and separated into two chambers) by the horizontal heat insulation member 31 and the vertical heat insulation member 33. In other words, the air layer 20 in the jacket structure of the thermostatic apparatus 1 is made compact. This can reduce the occurrence of temperature unevenness inside the constant temperature chamber 10 due to the differences in temperature rise time between portions of the air jacket structure and the like.

As illustrated in Figs. 1, 3, etc., the separator including the horizontal heat insulation member 31 and the vertical heat insulation member 33 is arranged inside the outer tank 11. The horizontal heat insulation member 31 is arranged at substantially the center position of a top surface portion of the outer tank 11 in the right-left direction, as illustrated in Fig. 2(a), for example, and is arranged so as to extend in the front-rear direction of the body 3 (or the outer tank 11), as illustrated in Figs. 1 and 3. As illustrated in Fig. 3, for example, the vertical heat insulation member 33 is connected to or continuous with the horizontal heat insulation member 31 and is arranged at substantially the center position on the rear portion (back portion) of the outer tank 11 in the right-left direction so as to extend along the up-down direction of the body 3 (or the outer tank 11).

As illustrated in Fig. 2(b), for example, the horizontal heat insulation member 31 has a first horizontal heat insulation member 31a and a second horizontal heat insulation member 31b. The first horizontal heat insulation member 31a and the second horizontal heat insulation member 31b are arranged such that the first portion 14, at which the opposite end portions 13a and 13b of the thin plate of stainless steel forming the inner tank 13, is sandwiched between the first horizontal heat insulation member 31a and the second horizontal heat insulation member 31b.

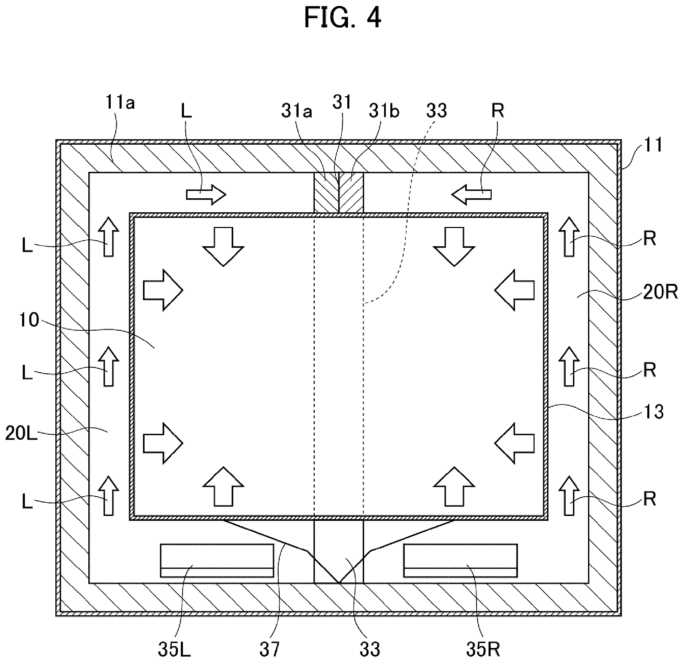

Incidentally, as illustrated in Fig. 4, for example, the inner surface of the outer tank 11 (the surface facing the air layer 20 and the inner tank 13) is covered with an outer-tank heat insulation material 11a. The outer surfaces of the horizontal heat insulation member 31 and the vertical heat insulation member 33 (the surfaces on the outer tank 11 side) are arranged in contact with the outer-tank heat insulation material 11a. In this way, the air layer 20 on the outer side of the inner tank 13 is equally separated into the left air layer 20L and the right air layer 20R.

Moreover, as illustrated in Fig. 4, for example, the two heaters (heating unit) 35L and 35R as thermostatic units are arranged on the lower side of the inner tank 13 in the outer tank 11. The two heaters 35L and 35R are arranged with a prescribed distance left therebetween in the right-left direction for the left air layer 20L and the right air layer 20R, respectively. Also, on the lower side of the inner tank 13 in the outer tank 11, a flow division plate 37 is arranged which guides air (thermostatic medium) L and air (thermostatic medium) R heated (temperature-controlled) by the two heaters 35L and 35R to the left air layer 20L and the right air layer 20R, respectively.

Here, Fig. 4 is a schematic view illustrating a heating method of the thermostatic apparatus 1 according to the present embodiment.

As illustrated in Fig. 4, air heated by the heater 35L is guided to the left air layer 20L by the flow division plate 37 as indicated by arrows L illustrated in the figure. Moreover, air heated by the heater 35R is guided to the right air layer 20R by the flow division plate 37 as indicated by arrows R illustrated in the figure. As a result, the left half of the constant temperature chamber 10 is heated by the air L heated by the heater 35L and guided to the left air layer 20L. Moreover, the right half of the constant temperature chamber 10 is heated by the air R heated by the heater 35R and guided to the right air layer 20R.

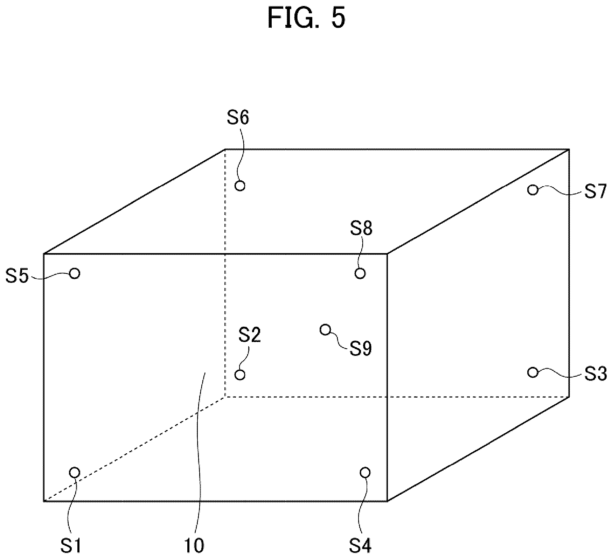

Fig. 5 is a schematic view illustrating a temperature detection method of the thermostatic apparatus 1 according to the present embodiment.

In the present embodiment, as illustrated in Fig. 5, for example, temperature detection sensors S1 to S9 are arranged at nine points inside the constant temperature chamber 10. The temperature change at each point at the time of heating was tested. Note that the above nine points were four points on lower front and rear sides of the constant temperature chamber 10, four points on upper front and rear sides, and one point in the center.

Fig. 6 illustrates the test results in a comparative fashion. Fig. 6(a) is a graph exemplarily illustrating a result of temperature detection of the thermostatic apparatus 1 according to the present embodiment. Fig. 6(b) is a graph exemplarily illustrating a result of temperature detection of a common natural convection-type thermostat (not illustrated). Note that Figs. 6(a) and 6(b) are graphs with a horizontal axis representing a time (t) axis and a vertical axis representing temperatures (°C). In Figs. 6(a) and 6(b), information of the temperatures in the constant temperature chamber 10 detected by the sensors S1 to S9 is illustrated in chronological order.

The test was carried out by setting the set temperature of the constant temperature chamber 10 to about 58.0°C and repetitively controlling the heaters 35L and 35R at regular time intervals. Note that, in this test, the heaters 35L and 35R were repetitively turned on and off at intervals of 3 hours. Then, the temperatures detected by the sensors S1 to S9 were checked for a temperature shift and a trend change (distortion phenomenon).

The test result for the conventional apparatus indicates the occurrence of a temperature shift (A) in which the stabilized temperatures shifted by about 1 to 2°C, as illustrated in Fig. 6(b), and the occurrence of a temperature distribution distortion phenomenon, such as a trend change in which the output of the sensor S2 became smaller than the output of the sensor S9, as illustrated in Fig. 7(b). In contrast, in the test using the thermostatic apparatus 1 according to the present embodiment, a temperature shift or a temperature distribution distortion phenomenon as the above was not confirmed, as illustrated in Fig. 7(a).

The thermostatic apparatus 1 according to the present embodiment has a configuration in which the air layer 20 inside the outer tank 11 is equally separated into the left air layer 20L and the right air layer 20R in the right-left direction. This configuration reduces the variation in the rate of temperature rise between portions of the air jacket at the time of heating even if the ambient (outside) temperature at the installed location is unstable. It is therefore possible to reduce the occurrence of temperature unevenness inside the inner tank 13 attributable to the power-on timing, the differences in temperature rise time between portions of the air jacket due to the structure of the jacket, and the like, and also stabilizing the temperature inside the constant temperature chamber 10 in a reproducible manner.

As described above, in the thermostatic apparatus 1 in the present embodiment, the separator including the horizontal heat insulation member 31 and the vertical heat insulation member 33 divides and separates the air layer 20 in the jacket structure into the laterally symmetrical left air layer 20L and right air layer 20R. With this configuration, the air layers 20L and 20R are more compact than the air layer 20 before being separated. These compact left air layer 20L and right air layer 20R are provided with the air L and the air R which have been individually heated. This makes it possible to provide a structure that is not easily affected by the environment temperature around the thermostatic apparatus 1 and the like. It is therefore possible to reduce the occurrence of temperature unevenness inside the inner tank attributable to the power-on timing, the differences in temperature rise time between portions of the air jacket due to the structure of the air jacket, and the like to reduce the temperature shift and the temperature distribution change.

In particular, temperature unevenness inside the constant temperature chamber 10 can be reduced without providing a fan or the like. Accordingly, disadvantages such as an increase in manufacturing cost, vibrations, and a deterioration in temperature gradient will not occur.

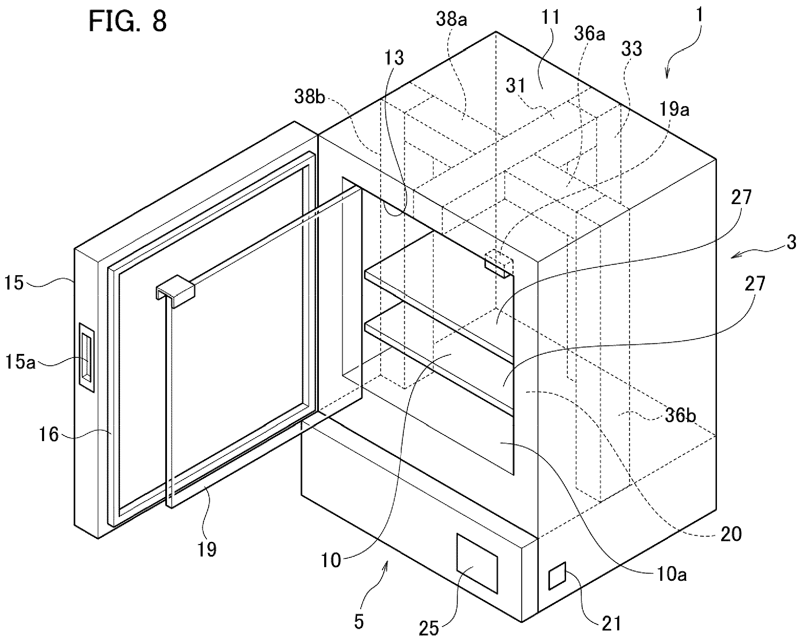

A structure in which the outer tank 11 is equally separated into the left air layer 20L and the right air layer 20R in the right-left direction has been described as the thermostatic apparatus 1 in the embodiment. However, the jacket structure of the thermostatic apparatus is not limited to this. For example, as illustrated in Fig. 8, the air layer 20 in the jacket structure inside the outer tank 11 may be separated into four parts (separated into four chambers) by horizontal heat insulation members 31, 36a, and 38a and vertical heat insulation members 33, 36b, and 38b, and these four separated layers may be individually heated.

Note that the number of air layers or chambers into which to separate the air layer 20 is not limited, as a matter of course.

In the embodiment, the configuration of the air jacket structure is such that the one end portion 13a of the inner tank 13 at the first portion 14 supports the outer tank 11, as illustrated in Fig. 2(b). However, the configuration of the air jacket structure is not limited to this. The present invention is applicable to various thermostatic apparatuses having air jacket structures (air jacket natural convection structures).

Also, the thermostatic medium that can be used in (supplied to) the jacket structure of the thermostatic apparatus 1 is not limited to air. The thermostatic medium in the jacket structure used in the thermostatic apparatus may be, for example, water, oil, antifreeze, gas, or the like.

Also, in the embodiment, the thermostatic apparatus 1 including the heaters 35L and 35R as heating units (temperature control units) has been exemplarily described. However, the present invention is applicable also to thermostatic apparatuses including cooling units as temperature control units, for example.

In any embodiments, the thermostatic apparatus 1 may include a humidistat that controls the humidity inside the inner tank 13, for example. This configuration can provide a thermostatic and humidistatic apparatus having a function of maintaining constant humidity to prevent condensation.

## Claims

1. A thermostatic apparatus (1) comprising:
an outer tank (11);
an inner tank (13) provided inside the outer tank (11) and forming a jacket structure with a space between the inner tank (13) and the outer tank (11);
a separator (31, 33) arranged inside the outer tank (11) and separating the jacket structure into a plurality of chambers (20L, 20R); and
a thermostatic unit (35L, 35R) configured to supply temperature-controlled thermostatic medium (L, R) to each of the plurality of chambers (20L, 20R) separated by the separator (31, 33),
**characterized in that**
the separator (31, 33) includes:
a horizontal heat insulation member (31) arranged at a center of a top surface portion of the outer tank (11) in a right-left direction and extending in a front-rear direction; and
a vertical heat insulation member (33) connected to the horizontal heat insulation member (31) and arranged at a center of a back portion of the outer tank (11) in the right-left direction and extending in an up-down direction.

2. The thermostatic apparatus (1) according to claim 1, further comprising:
a temperature detector (30) configured to detect temperature of the inner tank (13); and
a controller (24) configured to control the thermostatic unit (35L, 35R) based on the temperature detected by the temperature detector (30).

3. The thermostatic apparatus (1) according to claim 1 or 2, wherein the thermostatic unit (35L, 35R) is heating units arranged on a lower side of the inner tank (13) for each of the plurality of chambers (20L, 20R) in the outer tank (11).

4. The thermostatic apparatus (1) according to claim 1, further comprising a humidistat configured to control humidity inside the inner tank (13).

5. The thermostatic apparatus (1) according to claim 1, wherein
the inner tank (13) is formed of a thin plate folded into a rectangular shape,
opposite end portions (13a, 13b) of the thin plate are partly joined to each other at a center position on a top surface portion of the inner tank (13) in a right-left direction to form a first portion (14), and
an end portion of one of the opposite end portions (13a, 13b) at the first portion (14) extends toward the outer tank (11) and is folded in an L-shape to support the outer tank (11).

6. The thermostatic apparatus (1) according to claim 5, wherein
the horizontal heat insulation member (31) includes a first horizontal heat insulation member (31a) and a second horizontal heat insulation member (31b) arranged to sandwich the first portion (14) therebetween.

7. The thermostatic apparatus (1) according to any one of claims 1 to 5, further comprising a flow division plate (37) arranged on a lower side of the inner tank (13) and configured to guide the thermostatic medium (L, R) whose temperature is controlled by the thermostatic unit (35L, 35R).

8. A temperature distribution improvement method of improving a temperature distribution of an inner tank (13) of a thermostatic apparatus (1) including an outer tank (11) and the inner tank (13) provided inside the outer tank (11) and forming a jacket structure with a space between the inner tank (13) and the outer tank (11), the temperature distribution improvement method comprising:
separating the jacket structure into a plurality of chambers (20L, 20R) by providing a separator (31, 33) inside the outer tank (11); and
supplying thermostatic medium (L, R) whose temperature is controlled by a thermostatic unit (35L, 35R) to each of the plurality of chambers (20L, 20R) separated by the separator (31, 33),
**characterized in that**
the separator (31, 33) includes:
a horizontal heat insulation member (31) arranged at a center of a top surface portion of the outer tank (11) in a right-left direction and extending in a front-rear direction; and
a vertical heat insulation member (33) connected to the horizontal heat insulation member (31) and arranged at a center of a back portion of the outer tank (11) in the right-left direction and extending in an up-down direction.

## Patentansprüche

1. Eine Thermostatvorrichtung (1), aufweisend:
einen Außentank (11);
einen Innentank (13), der innerhalb des Außentanks (11) angeordnet ist und eine Mantelkonstruktion mit einem Zwischenraum zwischen dem Innentank (13) und dem Außentank (11) bildet;
einen Separator (31, 33), der im Inneren des Außentanks (11) angeordnet ist und die Mantelkonstruktion in eine Vielzahl von Kammern (20L, 20R) unterteilt; und
eine Thermostateinheit (35L, 35R), die so ausgelegt ist, dass sie temperaturgeregeltes Thermostatmedium (L, R) an jede der Vielzahl von Kammern (20L, 20R), die durch den Separator (31, 33) voneinander getrennt sind, zuführt
**dadurch gekennzeichnet, dass**
der Separator (31, 33) aufweist:
ein horizontales Wärmeisolierelement (31), das in einer Rechts-Links-Richtung in der Mitte eines oberen Oberflächenabschnitts des Außentanks (11) angeordnet ist und sich in einer Vorder-Hinter-Richtung erstreckt; und
ein vertikales Wärmeisolierelement (33), das mit dem horizontalen Wärmeisolierelement (31) verbunden ist und in der Rechts-Links-Richtung in der Mitte eines hinteren Abschnitts des Außentanks (11) angeordnet ist und sich in einer Auf-Ab-Richtung erstreckt.

2. Die Thermostatvorrichtung (1) nach Anspruch 1, weiter aufweisend:
einen Temperaturdetektor (30), der dazu ausgelegt ist, die Temperatur des Innentanks (13) zu erfassen; und
eine Steuereinheit (24), die dazu ausgelegt ist, die Thermostateinheit (35L, 35R) auf der Grundlage der vom Temperaturdetektor (30) erfassten Temperatur zu steuern.

3. Die Thermostatvorrichtung (1) nach Anspruch 1 oder 2, wobei die Thermostateinheit (35L, 35R) aus Heizeinheiten besteht, die an einer Unterseite des Innentanks (13) für jede der Vielzahl von Kammern (20L, 20R) im Außentank (11) angeordnet sind.

4. Die Thermostatvorrichtung (1) nach Anspruch 1, weiter aufweisend einen Hygrostat, der so konfiguriert ist, dass er die Luftfeuchtigkeit im Inneren des Innentanks (13) regelt.

5. Die Thermostatvorrichtung (1) nach Anspruch 1, wobei
der Innentank (13) aus einer dünnen Platte gebildet ist, die zu einer rechteckigen Form gefaltet ist,
gegenüberliegende Endabschnitte (13a, 13b) der dünnen Platte an einer mittleren Position auf einem oberen Oberflächenabschnitt des Innentanks (13) in einer Rechts-Links-Richtung teilweise miteinander verbunden sind, um einen ersten Abschnitt (14) zu bilden, und
sich ein Endabschnitt eines der gegenüberliegenden Endabschnitte (13a, 13b) am ersten Abschnitt (14) in Richtung des Außentanks (11) erstreckt und L-förmig gefaltet ist, um den Außentank (11) abzustützen.

6. Die Thermostatvorrichtung (1) nach Anspruch 5, wobei
das horizontale Wärmeisolierelement (31) ein erstes horizontales Isolierelement (31a) und ein zweites horizontales Isolierelement (31b) aufweist, die so angeordnet sind, dass sie den ersten Abschnitt (14) zwischen sich einschließen.

7. Die Thermostatvorrichtung (1) nach einem der Ansprüche 1 bis 5, weiter aufweisend eine Strömungsaufteilplatte (37), die an einer Unterseite des Innentanks (13) angeordnet und so ausgebildet ist, dass sie das Thermostatmedium (L, R) leitet, dessen Temperatur durch die Thermostateinheit (35L, 35R) geregelt wird.

8. Ein Verfahren zur Verbesserung der Temperaturverteilung zur Verbesserung einer Temperaturverteilung eines Innentanks (13) einer Thermostatvorrichtung (1), die einen Außentank (11) und den innerhalb des Außentanks (11) angeordneten Innentank (13) aufweist und eine Mantelstruktur mit einem Zwischenraum zwischen dem Innentank (13) und dem Außentank (11) bildet, wobei das Verfahren zur Verbesserung der Temperaturverteilung aufweist:
Unterteilen der Mantelstruktur in eine Vielzahl von Kammern (20L, 20R) durch Anordnen eines Separators (31, 33) im Inneren des Außentanks (11); und
Zuführen eines Thermostatmediums (L, R), dessen Temperatur durch eine Thermostateinheit (35L, 35R) geregelt wird, zu jeder der Vielzahl von Kammern (20L, 20R), die durch den Separator (31, 33) voneinander getrennt sind,
**dadurch gekennzeichnet, dass**
der Separator (31, 33) aufweist:
ein horizontales Wärmeisolierelement (31), das in einer Rechts-Links-Richtung in der Mitte eines oberen Oberflächenabschnitts des Außentanks (11) angeordnet ist und sich in einer Vorder-Hinter-Richtung erstreckt; und
ein vertikales Wärmeisolierelement (33), das mit dem horizontalen Wärmeisolierelement (31) verbunden ist und in der Rechts-Links-Richtung in der Mitte eines hinteren Abschnitts des Außentanks (11) angeordnet ist und sich in einer Auf-Ab-Richtung erstreckt.

## Revendications

1. Appareil thermostatique (1), comprenant :
une cuve externe (11) ;
une cuve interne (13) prévue à l'intérieur de la cuve externe (11) et formant une structure d'enveloppe avec un espace entre le tambour interne (13) et le tambour externe (11) ;
un séparateur (31, 33) disposé à l'intérieur de la cuve externe (11) et séparant la structure d'enveloppe en une pluralité de chambres (20L, 20R) ; et
une unité thermostatique (35L, 35R) configurée pour alimenter en milieu thermostatique à température commandée (L, R) chacune de la pluralité de chambres (20L, 20R) séparées par le séparateur (31, 33),
**caractérisé en ce que**
le séparateur (31, 33) inclut :
un élément d'isolation thermique horizontal (31) agencé au centre d'une partie de surface supérieure de la cuve externe (11) dans une direction droite-gauche et s'étendant dans une direction avant-arrière ; et
un élément d'isolation thermique vertical (33) connecté à l'élément d'isolation thermique horizontal (31) et agencé au centre d'une partie arrière de la cuve externe (11) dans la direction droite-gauche et s'étendant dans une direction haut-bas.

2. Appareil thermostatique (1) selon la revendication 1, comprenant en outre :
un détecteur de température (30) configuré pour détecter la température de la cuve interne (13) ; et
un dispositif de commande (24) configuré pour commander l'unité thermostatique (35L, 35R) sur la base de la température détectée par le détecteur de température (30).

3. Appareil thermostatique (1) selon la revendication 1 ou 2, dans lequel l'unité thermostatique (35L, 35R) est des unités de chauffage agencées sur un côté inférieur de la cuve interne (13) pour chacune de la pluralité de chambres (20L, 20R) dans la cuve externe (11).

4. Appareil thermostatique (1) selon la revendication 1, comprenant en outre un humidistat configuré pour commander l'humidité à l'intérieur de la cuve interne (13).

5. Appareil thermostatique (1) selon la revendication 1, dans lequel
la cuve interne (13) est formée d'une plaque mince pliée en une forme rectangulaire, des parties d'extrémité opposées (13a, 13b) de la plaque mince sont partiellement jointes l'une à l'autre en une position centrale sur une partie de surface supérieure de la cuve interne (13) dans une direction droite-gauche pour former une première partie (14), et
une partie d'extrémité de l'une des parties d'extrémité opposées (13a, 13b) au niveau de la première partie (14) s'étend vers la cuve externe (11) et est pliée en forme de L pour supporter la cuve externe (11).

6. Appareil thermostatique (1) selon la revendication 5, dans lequel
l'élément d'isolation thermique horizontal (31) inclut un premier élément d'isolation thermique horizontal (31a) et un second élément d'isolation thermique horizontal (31b) agencé pour enserrer la première partie (14) entre ceux-ci.

7. Appareil thermostatique (1) selon l'une quelconque des revendications 1 à 5, comprenant en outre une plaque de division d'écoulement (37) agencée sur un côté inférieur de la cuve interne (13) et configurée pour guider le milieu thermostatique (L, R) dont la température est commandée par l'unité thermostatique (35L, 35R).

8. Procédé d'amélioration de distribution de température pour améliorer une distribution de température d'une cuve interne (13) d'un appareil thermostatique (1) incluant une cuve externe (11) et la cuve interne (13) prévue à l'intérieur de la cuve externe (11) et formant une structure d'enveloppe avec un espace entre le tambour interne (13) et le tambour externe (11), le procédé d'amélioration de distribution de température comprenant les étapes consistant à :
séparer la structure d'enveloppe en une pluralité de chambres (20L, 20R) en prévoyant un séparateur (31, 33) à l'intérieur de la cuve externe (11) ; et
fournir un milieu thermostatique (L, R) dont la température est commandée par une unité thermostatique (35L, 35R) à chacune de la pluralité de chambres (20L, 20R) séparées par le séparateur (31, 33),
**caractérisé en ce que**
le séparateur (31, 33) inclut :
un élément d'isolation thermique horizontal (31) agencé au centre d'une partie de surface supérieure de la cuve externe (11) dans une direction droite-gauche et s'étendant dans une direction avant-arrière ; et
un élément d'isolation thermique vertical (33) connecté à l'élément d'isolation thermique horizontal (31) et agencé au centre d'une partie arrière de la cuve externe (11) dans la direction droite-gauche et s'étendant dans une direction haut-bas.
